# EUROPEAN PATENT SPECIFICATION

(11) **EP 4 323 049 B1**
(45) Date of publication and mention of the grant of the patent: **28.05.2025**
(21) Application number: 22723128.9
(22) Date of filing: 15.04.2022
(51) Int. Cl.: A61M 35/00

(54) **UNDERARM SPRAY DELIVERY DEVICE**
UNTERARMSPRAYVERABREICHUNGSVORRICHTUNG
DISPOSITIF D'ADMINISTRATION DE PULVÉRISATION SOUS L'AISSELLE

(30) Priority: 15.04.2021 EP 21168582
(43) Date of publication of application: 21.02.2024
(73) Proprietor: Xero Pharmaceuticals IP B.V., 1796 AB De Koog (NL)
(72) Inventor: LENEMAN, Marijn Jacob, 8014 DE Zwolle (NL); VENEMA, Aalt Willem, 8901 VV Zuidhorn (NL); HOMAN, Berend, 9458 TB Balloo (NL)
(74) Representative: Nederlandsch Octrooibureau
(86) International application number: PCT/EP2022/060164
(87) International publication number: WO 2022/219177

(56) References cited:
- US-A1- 2004 050 964
- US-A1- 2016 243 345

## Description

### Field of the invention

The present invention relates to a hand-held underarm spray delivery device for topically delivering a medicinal spray to a person's armpit, wherein the delivery device comprises a shroud for preventing the spray from reaching areas other than the person's armpit. Typically, medicinal spray, which may for instance comprise an aqueous formulation comprising buffered tiotropium bromide, or ipratropium, for topical treatment of hyperhidrosis, should only be applied to the person's armpit, and contact of the spray with any other part of the person, e.g. his or her fingers, and/or inhalation of the spray is to be avoided.

### Background art

From US 2004/050964 A a hand-held dispensing device is known for dispensing and applying a substance, such as a medicinal spray, to the skin of a host. The known device includes a hollow body, a capsule mounted within the hollow body for containing the substance, a nozzle mounted within the hollow body communicating with the substance in the capsule, an actuator to cause metered quantities of the substance to be dispensed from the capsule through the nozzle, a shroud defining an exit space for receiving the substance emerging from the nozzle, and a cap detachably mounted on the shroud to selectively open and close the nozzle and thereby control escape of the substance from the capsule.

However, the known device is somewhat unwieldy to use and carry around. It is an object of the invention to provide an underarm spray delivery device which at least partially overcomes this drawback.

### Summary of the invention

According to this end, the present invention provides a hand-held underarm spray delivery device, comprising: a container for containing medically active ingredient; an actuatable pump connected to the container and comprising a spray dispensing orifice with a spray axis, wherein the pump is adapted for, upon actuation, releasing a spray comprising the medically active ingredient through said orifice along the spray axis; a housing comprising a first part, a second part, and a flexible shroud attached to the first and the second part, wherein the pump is arranged within the housing, and wherein the first part and/or the second part is provided with an opening aligned with the spray dispensing orifice for allowing spray released through the dispensing orifice to pass through, wherein the second part is rotatable about an axis of rotation relative to the first part between an open position in which spray can pass out of the housing, and a closed position in which spray is prevented from passing out of the housing. When the second part is in the closed position, the device takes up relatively little space, in this manner providing a device that can easily be carried in a purse or pocket.

Typically, the first and second housing part are preferably substantially rigid, e.g. made from, or comprising, a hard plastic material such as polypropylene, polyethylene, an acetal copolymer, and the shroud is typically elastic, e.g. made from, or comprising, an elastic material such as silicone or rubber. The substantially rigid first and second part provide a protective shell for the pump and part or all of the container against deformation and damage. When the second part is in the open position, inner sides of the flexible shroud and of the first and second part together substantially prevent passage of spray from said inner side to an outer side of the shroud other than beyond the edge of the shroud. When the second part is in the closed position, interior surfaces of the device that may come in contact with the spray are completely covered, and the device can be picked up and held without risk of coming into contact with the spray.

In an embodiment the housing, when the second part is in the closed position, seals off an interior volume delimited by the flexible shroud and facing inner surfaces of the first and second housing part, in a substantially liquid tight manner. Liquid containing the medically active ingredient is thus prevented from passing out of the interior volume when the device is closed.

In an embodiment the first and/or second part of the housing is provided with one or more locking mechanisms, adapted for locking the first and second part in the closed position. A user will thus actively have to open the device before the device is ready to dispense spray. Additionally, when the shroud, or at least the circumferential distal edge of the shroud, comprises an elastic material, the locking mechanisms help to press the first part and the second part together such that the shroud can form a liquid-tight seal. The one or more locking mechanisms, which may be spring loaded locking mechanisms, are preferably arranged to be operated by a user from the outer side of the housing when the housing is in the closed position.

In an embodiment the device further comprises a biasing element for biasing the second part to the open position. In this manner, in order to open the device, a user does not have to touch the distal edge of the shroud with his or her fingers. This further helps the user to avoid contact between the user's fingers and any spray that may be present along the distal edge of the shroud. In case the device is provided with one or more locking mechanisms, releasing the locking mechanism(s) will typically result in the second part moving to the open position.

In an embodiment, the pump is adapted for providing a metal-contact free fluid path for the spray through the pump upon actuation of the pump. A suitable pump is offered by Aptar Pharma under the name "Advanced Preservative Free plus".

In an embodiment the first part and the second part each have an inner surface, wherein a layer of liquid absorbing material is provided on the inner surface of the first and/or second part, for preventing liquid containing medically active ingredient from running along the inner surface of the first and/or second part out of the device. An example of absorbing material is tissue. When some of the spray, rather than reaching its intended target, is directed onto the absorbing material, it may be thus substantially prevented that the medically active ingredient will leak out of the device.

In an embodiment the pump is adapted to be actuated by pressing the container relative to the first and second housing part towards the spray dispensing orifice, preferably by pressing the container towards said orifice along the axis of rotation. Thus, when the pump is actuated, the position of the orifice relative to the first and second part of the housing remains the same.

In an embodiment the container comprises a circumferential portion arranged within the housing, and an end surface spaced distally from the dispensing orifice and arranged to be pressed towards the spray dispensing orifice by a user for actuating the pump. In this manner, the pump can be operated by pressing the end surface of the container towards the orifice. Preferably the end surface of the container is arranged outside of the first and housing parts at least when the pump is not actuated and preferably also when the pump is actuated. The end surface of the container may in this manner function as a button, or part of such a button, for actuating the pump.

In an embodiment the circumferential portion of the container comprises a transparent material, and the first and/or second part of the housing comprises a see-through portion for allowing visual inspection, through the see-through portion and the transparent material, of a level of liquid in the container when the second part of the housing is in the closed position. Though the see-through portion may simply be formed as an opening in the first or second part of the housing, it is preferred that the see-through portion comprises a transparent piece of plastic material.

In an embodiment the flexible shroud comprises a circumferential distal edge shaped and adapted for lying against an armpit along said entire edge when the second part is in the open position. As the edge is adapted for lying against a user's armpit, escape of liquid through gaps between the user's skin and the shroud may be substantially avoided. The distal edge is preferably adapted for folding onto itself when the second part of the housing is in the closed position, for sealing an interior space defined by the housing. This may be achieved for instance when the distal edge of the flexible shroud comprises an elastic material that is adapted to be compressed between the first part and the second part of the housing when the second part is in the closed position.

In an embodiment, when seen in projection onto a plane normal to the spray axis and parallel to the axis of rotation, and when the second part is in the open position, each part of the circumferential distal edge is spaced apart from the spray axis by at least 3 cm. In this manner a spray cone is possible which substantially does not contact the first or second housing parts.

In an embodiment the device further comprises a blocking mechanism adapted for preventing actuation of the pump when the second part is in the closed position. In this manner, accidental spraying of the medically active ingredient is substantially avoided, allowing the device to be safely carried in a user's purse or pocket. The blocking mechanism preferably is arranged within the first and/or second part, to prevent a user from accidentally unblocking the mechanism.

In an embodiment the pump is releasably arranged within the housing, to allow the pump and/or the pump and container connected thereto to be replaced. In this embodiment the device is provided with a latch which is moveable between a first position in which the latch block movement of the pump out of the housing, and a second position in which the pump can be removed from the housing in a direction parallel to the axis of rotation, wherein the latch is adapted to be moveable from the first to the second position only when the second part of the housing is in the closed position. The latch is preferably arranged to be operated by a user from the outer side of the housing.

In an embodiment the axis of rotation is substantially parallel to the longitudinal axis of the container, preferably wherein the axis of rotation and the longitudinal axis of the container substantially coincide.

In an embodiment the spray axis is substantially perpendicular to the longitudinal axis of the container.

In an embodiment, when the second part of the housing is in the closed position the device has a substantially wedge-like shape, e.g. similar to the shape of a closed cockle, providing a particularly compact device.

In an embodiment the first and second housing part each comprise a distal edge, wherein when the second part is in the closed position the distal edges of the first and second part are spaced apart by a first distance, and when the second part is in the open position the distal edges of the first and second part are spaced apart by a second distance greater than the first distance. The first distance may correspond substantially to twice the thickness of the circumferential edge of the shroud when the second part is in the closed position, in which case the second distance is at least 3 times greater than the first distance.

Preferably, when seen in projection onto a plane normal to the axis of rotation, the container has an outer diameter which is greater than the first distance and smaller than the second distance.

In an embodiment the second part is adapted for rotating between the closed and the open position relative to the first part, and preferably also relative to the spray axis over an angle of between 65 and 80 degrees around the axis or rotation.

In an embodiment, when the second part is in the closed position, the entire device fits within a rectangular box of dimensions 12 cm x 12 cm x 3,3 cm.

In an embodiment the container is releasably attached to the pump by means of a liquid tight connection, e.g. a liquid-tight screw connection. By replacing an empty container with a container that contains a medically active component, the device can be reused many times.

In an embodiment the container contains an aqueous formulation comprising an anticholinergic agent as the active ingredient, such as tiotropium bromide, ipratropium, glycopyrronium, and/or oxybutynin. Besides affecting the sweat glands, some anticholinergic agents have the capability of influencing the gastrointestinal tract, urinary tract, lungs or other parts of the body. The dispensing device according to the invention helps prevent that the anticholinergic agent comes into contact with other parts of the user's body than the user's armpits, and in particular prevents the spray from being ingested or inhaled.

The formulation may further comprise an aqueous phase, and a dermatologically acceptable pH adjustment agent to provide the formulation with a pH in the range of 3.0 to 6.0 at 21°C. These compounds are highly suitable for topical treatment of hyperhidrosis. The pH of the aqueous formulation according to the invention is measured at 21°C using conventional techniques known to the skilled person.

The term "dermatologically acceptable pH adjustment agent" as used herein refers to buffering agents. The pH of the formulation is suitably adjusted by adding a dermatologically acceptable pH adjustment agent selected from buffering systems or salts of weak organic or inorganic acids, such as carbonate buffers, citrate buffers, phosphate buffers, acetate buffers, hydrochloric acid, lactic acid, tartric acid, diethylamine, triethylamine, diisopropylamine, aminomethylamine, or the like. Preferably, the buffer is citrate buffer. Typical citrate buffers are comprised of citric and sodium or potassium citrate, or citric acid and Na₂HPO₄.

The aqueous formulation may comprise dermatologically acceptable excipients. Typically, the excipients are selected from bactericides, preservatives, antioxidants, humectants, sequestering agents, moisturizers, emollients, surfactant, drying agents, fragrances and the like.

In an embodiment the aqueous formulation comprises 70-99.9 wt.% aqueous phase, by weight of the total formulation, preferably 80-99.7 wt.%, even more preferably 85-99.5, most preferably 90-99.25 wt.% aqueous phase by weight of the total formulation. According to a preferred embodiment, the aqueous phase comprises, based on the weight of the aqueous phase, at least 50 wt.% water, preferably at least 60 wt.% water, more preferably at least 75 wt.% water, even more preferably at least 80 wt.% water, most preferably at least 90 wt.% water. The term "aqueous phase" as used herein refers to an aqueous phase comprising water or pharmaceutically acceptable water-soluble components, such as ethanol, propylene glycol, glycerol, and conventional water-soluble components. The "aqueous phase" is in the liquid state or in the semi-solid state at 20°C, preferably the liquid state. All "wt.%" referred to herein are based on weight of total aqueous formulation, unless otherwise indicated.

In an embodiment the anticholinergic agent is "oxybutynin". "Oxybutynin equivalent" as used herein refers to oxybutynin free base or a dermatologically acceptable salt of oxybutynin, such as oxybutynin hydrochloride (C₂₂H₃₁NO₃.HCl; 4- diethylaminobut-2-ynylalpha-cyclohexylmandelate hydrochloride), or mixtures thereof. Oxybutynin as used herein refers to the (R) and (S) tereoisomers of oxybutynin, and mixtures of the stereoisomers. Preferably, oxybutynin equivalent is present in an aqueous formulation in an amount of 1.0 - 8 wt.% oxybutynin equivalent, more preferably 1.5 - 6.0 wt.%, more preferably in an amount of 2.0 - 5.0 wt.%, even more preferably 2.5 to 4.5 wt.%, based on total weight of the aqueous formulation. Suitable oxybutynin salts are selected from the group consisting of, but not limited to, acetate, bitartrate, citrate, edetate, edisylate, estolate, esylate, fumarate, gluceptate, gluconate, glutamate, hydrobromide, hydrochloride, lactate, malate, maleate, mandelate, mesylate, methylnitrate, mucate, napsylate, nitrate, pamoate, pantothenate, phosphate, salicylate, stearate, succinate, sulfate, tarmate, tartrate, xinafoate, palmitate, pamoic salt, a resonate salt, a laurate salt and others. Pharmaceutical derivatives of oxybutynin which are closely related to oxybutynin are also understood to fall within the scope of the present invention. Preferably, oxybutynin is oxybutynin hydrochloride or used in equivalent amount thereof.

In an embodiment the anticholinergic agent is "glycopyrronium". "Glycopyrronium equivalent" as used herein refers to glycopyrronium free base or a dermatologically acceptable salt of glycopyrronium, such as glycopyrronium bromide (C19H28BrNO3; [(3S)-1,1-dimethylpyrrolidin-1-ium-3-yl] (2R)-2-cyclopentyl-2-hydroxy-2-phenylacetate;bromide), or mixtures thereof. Preferably, glycopyrronium equivalent is present in an aqueous formulation in an amount of 0.25 - 6 wt.% glycopyrronium equivalent, more preferably 0.25 - 3.0 wt.%, even more preferably in an amount of 0.5 - 3.0 wt.%, yet more preferably 1.0 to 2.0 wt.%, based on total weight of the aqueous formulation. Suitable glycopyrronium salts are selected from the group consisting of, but not limited to, such as iodide, acetate , sulphate, chloride, fluoride, iodide, nitrate, sulfonate, phosphate, propionate, glycolate, pyruvate, oxalate, succinate, fumarate, tartrate, citrate, benzoate, methanesulfonate, 4-methylbenzenesulfonate (tosylate), salicylate and others. Pharmaceutical derivatives of glycopyrronium which are closely related to glycopyrronium are also understood to fall within the scope of the present invention. Preferably, glycopyrronium is glycopyrronium bromide or used in equivalent amount thereof.

In a preferred embodiment, the aqueous formulation comprises, by weight of the total formulation: 1.0 - 2.0 wt.% glycopyrronium equivalent; 70 - 98 wt.% aqueous phase; dermatologically acceptable pH adjustment agent to provide the formulation with a pH in the range of 3.0 to 6.0 at 21°C.

In an embodiment, the container contains an aqueous formulation for use in the treatment of a skin disease, said treatment comprising topically administering to a human patient an aqueous formulation comprising, by weight of the total formulation: 1.0 - 2.0 wt.% glycopyrronium equivalent; 70 - 98 wt.% aqueous phase; dermatologically acceptable pH adjustment agent comprising citrate, wherein the formulation has a pH in the range of 3.0 to 6.0, preferably 3.0 to 4.0 at 21°C. The skin disease may for instance be primary or secondary hyperhidrosis.

In an embodiment the anticholinergic agent is "ipratropium". "Ipratropium equivalent" as used herein refers to the substance (8-methyl-8-propan-2-yl-8- azoniabicyclo[3.2.1]octan-3-yl) 3-hydroxy-2-phenylpropanoate. Typically, ipratropium is present in the aqueous formulation in the form of a pharmaceutically acceptable salt. The pharmaceutically acceptable salt is suitably selected from ipratropium bromide anhydrous or monohydrate, or ipratropium chloride. Preferably, ipratropium is ipratropium bromide, more preferably ipratropium bromide monohydrate. Preferably, ipratropium as used herein refers to anhydrous or monohydrate ipratropium bromide. Typically, monohydrate ipratropium bromide is freely soluble in water (10 mg/mL) at 20 °C. Preferably, ipratropium equivalent is present in the aqueous formulation in an amount of 0.01 - 2 wt.% , more preferably 0.3 - 1.5 wt.%, even more preferably in an amount of 0.5 to 1.0 wt.%.

In a preferred embodiment, the aqueous formulation comprises, by weight of the total formulation: 0.01 - 2 wt.% ipratropium equivalent; 70 - 98 wt.% aqueous phase; dermatologically acceptable pH adjustment agent to provide the formulation with a pH in the range of 3.0 to 6.0 at 21°C. The skin disease may for instance be primary or secondary hyperhidrosis.

In an embodiment the anticholinergic agent is "tiotropium". "Tiotropium equivalent" as used herein refers to the substance (1α, 2β, 4β, 5α, 7β)-7- [(hydroxydi-2-thienylacetyl)oxy]-9,9-dimethyl-3-oxa-9-azoniatricyclo[3.3.1.02,4]nonane and salts thereof. Suitable salts are fluoride, chloride, bromide, iodide, C1-C4alkyl sulfate, sulfate, hydrogen sulfate, phosphate, hydrogen phosphate, dihydrogen phosphate, nitrate, maleate, acetate, trifluoroacetate, citrate, fumarate, tartrate, oxalate, succinate and benzoate. Preferably, tiotropium as used herein refers to anhydrous or monohydrate tiotropium bromide. Tiotropium equivalent, preferably tiotropium bromide is present in the aqueous formulation in an amount of 0.0001 - 1.5 wt.%, preferably in an amount of 0.001 - 1.0 wt.%, more preferably 0.01 - 1.0 wt.%, even more preferably 0.025 - 1.0 wt.%, yet more preferably 0.05 to 0.5 wt.%. Preferably, the aqueous formulation comprises 0.001 - 1.0 wt.% tiotropium equivalent, more preferably 0.01 - 1.0 wt.%, even more preferably 0.025 to 0.05 wt.% tiotropium equivalent, preferably tiotropium bromide. Typically, tiotropium equivalent is present in the aqueous formulation in the form of a pharmaceutically acceptable salt. The pharmaceutically acceptable salt is selected from the group consisting of fluoride, chloride, bromide, iodide, C1-C4alkyl sulfate, sulfate, hydrogen sulfate, phosphate, hydrogen phosphate, dihydrogen phosphate, nitrate, maleate, acetate, trifluoroacetate, citrate, fumarate, tartrate, oxalate, succinate and benzoate. The tiotropium equivalent maybe anhydrous or a monohydrate. Preferably, the tiotropium equivalent is a monohydrate. More preferably, the tiotropium equivalent is tiotropium bromide monohydrate, or tiotropium chloride monohydrate, most preferably tiotropium bromide monohydrate.

In a preferred embodiment, the container contains an aqueous formulation for use in the treatment of a skin disease, said treatment comprising topically administering to a human patient an aqueous formulation comprising, by weight of the total formulation: 0.0001 - 1.5 wt.% tiotropium equivalent; 70 - 99.9 wt.% aqueous phase; dermatologically acceptable pH adjustment agent comprising citrate, wherein the formulation has a pH in the range of 3.0 to 6.0, preferably 3.0 to 5.5 at 21°C. The skin disease may for instance be primary or secondary hyperhidrosis.

### Short description of drawings

The present invention will be discussed in more detail below, with reference to the attached drawings, in which
Fig. 1A shows an isometric view of underarm spray delivery device according to the present invention in an open;
Fig. 1B shows a rear side isometric view of the underarm spray delivery device of Fig. 1A, in a closed position;
Fig. 1C shows an exploded view of the device of Fig. 1A;
Figs. 1D and 1E illustrate a blocking mechanism for preventing actuation of the pump when the second housing portion is in the closed position;
Fig. 1F shows a detail of a latch for preventing the pump from being moved out of the housing when the second housing portion is in the open position;
Figs. 2A and 2B show side views of the device of Fig. 1A, respectively in an open position and a closed position; and
Fig. 2C shows a front view of the device of Fig. 1A, in the open position.

### Description of embodiments

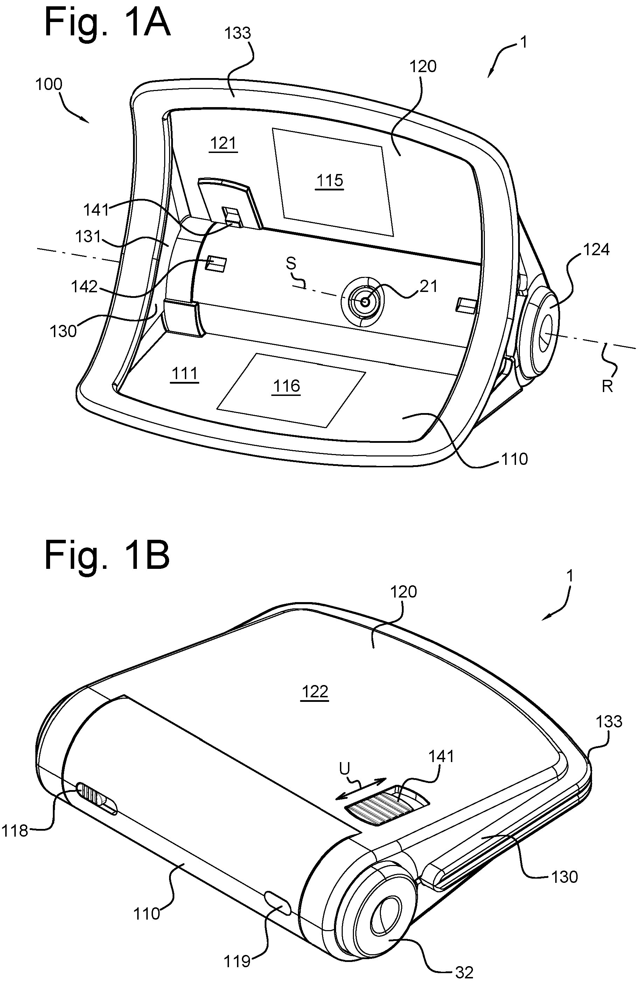

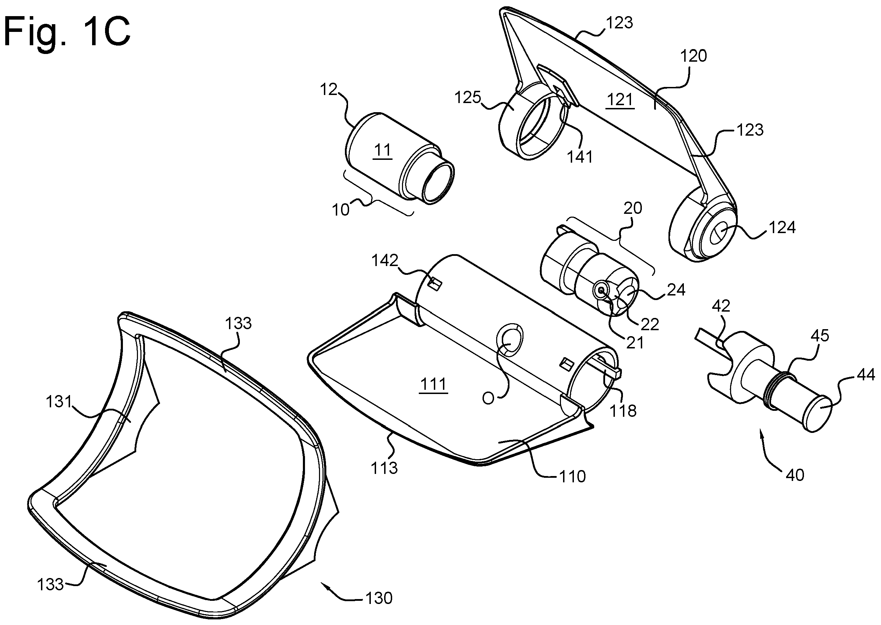

Figs. 1A and 1B respectively show an isometric front view and an isometric back view of an underarm spray delivery device 1 according to the invention in an open and in a closed position. Fig. 1C shows an exploded view of the same device in the open position. The underarm spray delivery device 1 comprises a housing 100 enveloping a pump 20 (shown in Fig. 1C) that is in fluid connection with a container 10 (also shown in Fig. 1C) which contains a medically active ingredient. The medically active ingredient may be for treating hyperhidrosis, and is typically intended to be sprayed, via spray dispensing orifice 21 of the pump 20 along its spray axis S, only onto a person's underarm or armpit. Contact with other parts of the person's body, and especially inhalation of the medically active ingredient, is generally to be avoided. The housing 100 comprises a first part 110 and a second part 120 hingeably connected to the first part 110, as well as a flexible shroud 130 which is attached to both the first part 110 and the second part and which has a circumferential distal edge 133. The second part 120 is hingeable around axis of rotation R relative to the first part 110 between an open position, shown in Fig. 1A and a closed position, shown in Fig. 1B. When in the open position, a person can place the shroud 130 with its circumferential distal edge 133 against his or her armpit over an intended area of skin that is to be treated with spray. In this manner a volume defined by the area of skin, the shroud and the first and second part is substantially sealed against escape of spray, so that inadvertent inhalation of the spray and contact of the spray with skin other than the intended area of skin may substantially be avoided. Inner surfaces 111, 121 of the first and second part are partially covered with corresponding layers 115,116 of liquid absorbing material.

The first housing part 110 and second housing part 120 each comprise a distal edge 113, 123 in contact with the circumferential distal edge of the shroud 130 is

The device 1 can be folded such that the second part 120 is rotated relative to the first part 110 around the axis of rotation R from the closed position to the open position or vice versa, by a person touching only the exterior surfaces of the housing. During regular use, a person's fingers do not have to come into contact with inner surfaces 111, 121 and 131 of the first and second part and the shroud, which face a side of the device where spray may adhere, i.e. a "wet side" of the device.

Fig. 1C shows an exploded view of the device 1 of Figs. 1A and 1B, in which the container 10 and pump 20 are more clearly visible. The container has a circumferential portion 11 that is proximal to the spray orifice 21, and an end surface 12 at the opposite distal end of the container. When in the assembled state show in Figs. 1A and 1B, a body 40 surrounds at least a portion of the pump 20 on a side of the pump facing away from the container 10. The body 40 is provided with a recessed edge 42 for partially abutting and accommodating therein an outer surface of nozzle 22 in which the spray orifice 21 is arranged, so that, in the assembled state, the pump 20 is rotationally fixed with respect to the body 40, and end surface 44 of the body abuts end surface 124 of the second part. In this manner the end surface 44 of the body 40 as well as the end surface 24 of the pump that is in contact therewith, are prevented from sliding out of the device at the side of end surface 124, and it is ensured that the spray orifice 21 of the pump 20 remains aligned with the opening O of the first housing part 110.

When assembled, the container 10 is translatable relative to the first and second housing portion 110, 120 along the axis of rotation R, so that pressing end surface 12 of the container towards the pump 20 causes actuation of the pump so that the active ingredient is sprayed out of dispensing orifice 21.

In order to prevent the device, when closed, from inadvertently opening, the first and second housing portion 110 and 120 are provided with a locking mechanism 141, 142. When the device is in the closed position shown in Fig. 1B, sliding button 141, which is biased to the locked position shown in Fig.1B, has to be moved along direction U which is normal to the axis of rotation R, so that part of the button 141 is moved out of corresponding notch 142 in the first housing portion, in this manner allowing the second housing portion to be rotated to the open position shown in Fig. 1A.

As shown in Fig. 1B, the first housing part 110 is provided with a see-through portion window 119, which allows a view of transparent circumferential surface 11 of the container 10, so that a can easily see the extent to which the container 10 is filed with aqueous solution containing the medically active ingredient.

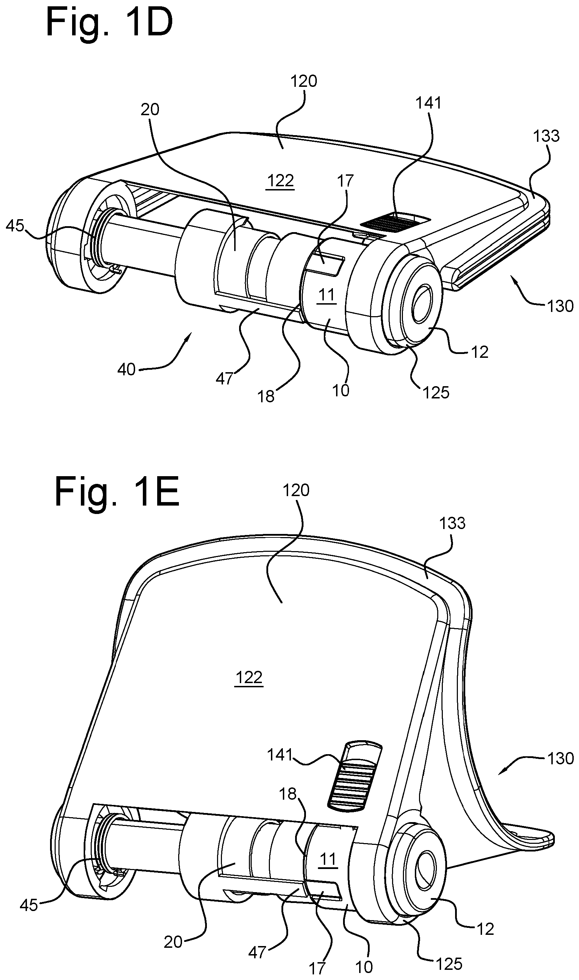

Figs. 1D and 1E show a detail of the same device, with the first housing portion omitted to show that the device is provided with a blocking mechanism 47,17 for preventing actuation of the pump when the second part 120 is in the closed position. The blocking mechanism comprises a groove 17 which extends in outer surface of the container 10 and parallel to the axis of rotation R. The blocking mechanism further comprises an protrusion 47 that is part of the body 40 and which is adapted for fitting in the groove 17. In Fig. 1D, the second portion 120 is shown in the closed position in which the distal edge 133 of shroud 130 is folded onto itself. In this position, the protrusion 47 is out of alignment with respect to the groove 17, with a distal end of the protrusion 47 contacting edge 18 of the container which blocks movement of the surface 12 of container 10 towards the spray orifice. inadvertent actuation of the pump when the device is in the closed position is thus prevented. Fig. 1E shows the device in the open position, in which the protrusion 47 is aligned with and partially inserted in the groove 17, allowing the surface 12 to be pressed towards the spray opening to actuate the pump 20.

Figs. 1D and 1E further show a spring 45 connected to both the first housing part 110 (not shown) and the second housing part, and biases the second housing part towards the open position. Thus, when sliding button 141 is moved out of the notch 142 (shown in Figs 1A and 1C), the device will open without the user having to touch the edge 133 of the shroud 130.

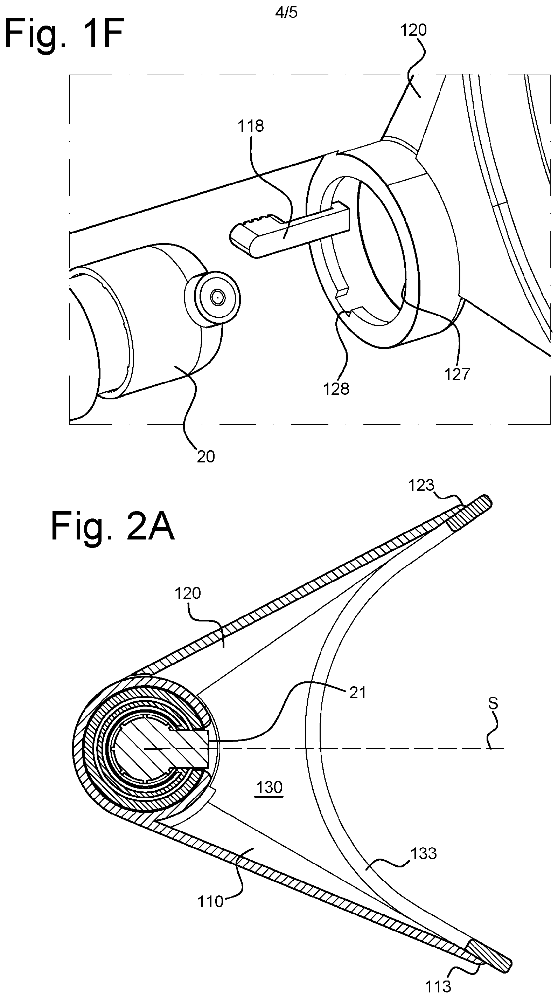

The pump and container are releasably arranged within the housing, allowing these to be replaced, e.g. to refill the container or replace an empty container with another container that is filled with medically active ingredient. During general use, though the container can slide within the housing, both the container and pump are prevented from sliding completely out of the housing. Fig. 1F illustrates how this may be achieved by means of a latch 118 which catches inward facing edge 127 of the second housing part when the second housing part is in the open position. The latch 118 is attached, e.g. directly or via body 40 - in particular via protrusion 47 of the body, to the pump 20 in such a manner that the latch 118 is translationally fixed with respect to the pump 20. When the second housing part 120 is in the open position shown, it catches against inward facing edge 127 of the second housing part in this manner preventing the pump 20 from sliding out of the housing. When the second housing part 120 is moved to the closed position, the latch 118 will align with recess 128 in the first housing part in such a manner that the latch no longer catches edge 127, allowing the pump can be removed from the housing, e.g.by pulling on the container and/or which gently pushing the latch radially inward and sliding the latch towards open end 125 of the second housing part 120, which open end 125 is of a dimension for allowing the container 12 and pump to pass therethrough. By ensuring that the pump and/or container can only be replaced and/or removed when the housing is closed, the risk of a user inadvertently coming into contact with medically active ingredient is further reduced.

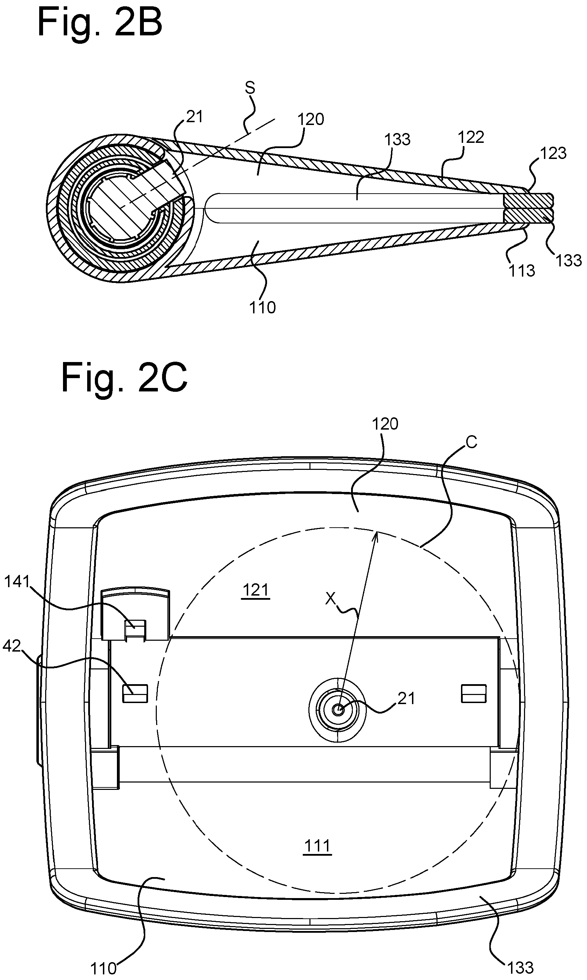

Figs. 2A and 2B show cross-sectional view of the underarm spray delivery device of Fig. 1A though a plane normal to the axis of rotation R and through the spray dispensing orifice 21, respectively in an open position and in a closed position. When in the open position shown in Fig. 2A, the curvature of the circumferential edge 133 allows the device to be placed with said edge against a person's armpit with the edge in contact with the person's skin along substantially the entire length of the edge. In the open position the spray axis S is substantially equidistant from the distal edges 113 and 123 of the first and second housing parts 110, 120. In the closed position shown in Fig. 2B, the spray axis S intersects the second housing part 120, and is located closer to the distal edge 123 of the second housing part than to the distal edge of the first housing part. Figs. 2A and 2B illustrate that upon opening and closing of the device, the second housing part moves with respect to the spray axis S, while the first housing part remains stationary with respect to the spray axis S.

Fig. 2C shows a front view of the device of the invention in the open position, in which a virtual circle C having radius X of 3,5 cm is schematically drawn. As can be seen, the spray axis S, which is shown here normal to the viewing direction, is spaced apart by at least the radius X from the circumferential edge 133 of the shroud 130.

The present invention has been described above with reference to a number of exemplary embodiments as shown in the drawings. Modifications and alternative implementations of some parts or elements are possible, and are included in the scope of protection as defined in the appended claims.

## Claims

1. A hand-held underarm spray delivery device (1), comprising:
a container (10) for containing medically active ingredient;
an actuatable pump (20) connected to the container and comprising a spray dispensing orifice (21) with a spray axis (S), wherein the pump is adapted for upon actuation releasing a spray comprising the medically active ingredient through said orifice along the spray axis;
a housing (100) comprising a first part (110), a second part (120), and a shroud (130) attached to the first and the second part, wherein the pump (20) is arranged within the housing (100), and wherein the first part and/or the second part is provided with an opening (O) aligned with the spray dispensing orifice for allowing spray released through the dispensing orifice to pass through,
**characterized in that** said shroud is flexible and **in that**
the second part (120) is rotatable about an axis of rotation (R) relative to the first part (110) between an open position in which spray can pass out of the housing, and a closed position in which spray is prevented from passing out of the housing.

2. Device according to claim 1, wherein the first part (110) and/or second part (120) of the housing is provided with one or more locking mechanisms (141, 142) adapted for locking the first and second part in the closed position, wherein said one or more locking mechanisms are arranged to be operated by a user from the outer side of the housing when the housing is in the closed position.

3. Device according to claim 1 or 2, further comprising a biasing element (45) for biasing the second part to the open position.

4. Device according to any one of the preceding claims, wherein the first part (110) has an inner surface (111) and wherein the second part (120) has an inner surface (121), wherein a layer of liquid absorbing material (115, 116) is provided on the inner surface (111, 121) of the first and/or second part, for preventing liquid containing medically active ingredient from running along the inner surface of the first and/or second part out of the device.

5. Device according to any one of the preceding claims, wherein the pump (20) is adapted to be actuated by pressing the container (10) relative to the first and second housing part towards the spray dispensing orifice (21), preferably by pressing the container towards said orifice along the axis of rotation.

6. Device according to any one of the preceding claims, wherein the container (10) comprises a circumferential portion (11) arranged within the housing, and an end surface (12) spaced distally from the dispensing orifice and arranged (12) to be pressed towards the spray dispensing orifice by a user for actuating the pump, preferably wherein the end surface is arranged outside of the housing.

7. Device according to claim 6, wherein the circumferential portion (11) of the container comprises a transparent material, and wherein the first and/or second part of the housing comprises a see-through portion (119) for allowing visual inspection, through the see-through portion and the transparent material, of a level of liquid in the container (10) when the housing (100) is in the closed position.

8. Device according to any one of the preceding claims, wherein the flexible shroud (131) comprises a circumferential distal edge (133) shaped and adapted for lying against an armpit along said entire edge when the second part is in the open position, preferably wherein said distal edge is adapted for folding onto itself when the second part is in the closed position, for sealing an interior space of the housing (100).

9. Device according to claim 8, wherein when seen in projection onto a plane normal to the spray axis (S) and parallel to the axis of rotation (R), and when the second part (120) is in the open position, each part of the circumferential distal edge (133) is spaced apart from the spray axis (S) by at least 3 cm.

10. Device according to any one of the preceding claims, further comprising a blocking mechanism (17, 47) adapted for preventing actuation of the pump (20) when the second part (120) is in the closed position.

11. Device according to any one of the preceding claims, wherein the pump (20) is releasably arranged within the housing (100), to allow the pump (20) and/or the pump and container (10) connected thereto to be replaced.

12. Device according to claim 11, comprising a operable latch (118) moveable between a first position in which the latch blocks movement of the pump (20) out of the housing, and a second position in which the pump can be removed from the housing in a direction parallel to the axis of rotation, wherein the latch (118) is adapted to be moveable from the first to the second position only when the housing is in the closed position.

13. Device according to any one of the preceding claims, wherein the container (10) contains an aqueous formulation a comprising an anticholinergic agent as the active ingredient, such as tiotropium bromide, ipratropium, glycopyrronium, and/or oxybutynin.

14. Device according to any one of the preceding claims, wherein the axis of rotation (R) is substantially parallel to the longitudinal axis of the container, preferably wherein the axis of rotation and the longitudinal axis of the container substantially coincide.

15. Device according to any one of the preceding claims, wherein the first housing part (110) and second housing part (120) each comprise a distal edge (113, 123), wherein when the second part is in the closed position the distal edges of the first and second part are spaced apart by a first distance and when the second part is in the open position the distal edges of the first and second part are spaced apart by a second distance greater than the first distance, preferably wherein, when seen in projection onto a plane normal to the axis of rotation, the container has an outer diameter which is greater than the first distance and smaller than the second distance.

## Patentansprüche

1. Tragbare Achselsprayliefervorrichtung (1), die aufweist:
einen Behälter (10) zum Enthalten eines medizinisch aktiven Inhaltsstoffs;
eine betätigbare Pumpe (20), die mit dem Behälter verbunden ist und eine Spraydispensieröffnung (21) mit einer Sprühachse (S) aufweist, wobei die Pumpe dazu angepasst ist, bei einer Betätigung ein Spray, das den medizinisch aktiven Inhaltsstoff aufweist, durch die Öffnung entlang der Sprühachse freizusetzen;
ein Gehäuse (100), das eine erste Partie (110), eine zweite Partie (120) und einen Kragen (130) aufweist, der an der ersten und der zweiten Partie angebracht ist, wobei die Pumpe (20) innerhalb des Gehäuses (100) angeordnet ist und wobei die erste Partie und/oder die zweite Partie mit einem Loch (O) versehen ist, das mit der Spraydispensieröffnung ausgerichtet ist, zu ermöglichen, dass ein Spray, das durch die Dispensieröffnung freigesetzt wird, hindurchtritt,
**dadurch gekennzeichnet, dass** der Kragen flexibel ist und dadurch, dass
die zweite Partie (120) um eine Drehachse (R) relativ zu der ersten Partie (110) zwischen einer offenen Position, bei der Spray aus dem Gehäuse treten kann, und einer geschlossenen Position drehbar ist, bei der Spray daran gehindert wird, aus dem Gehäuse zu treten.

2. Vorrichtung gemäß Anspruch 1, wobei die erste Partie (110) und/oder zweite Partie (120) des Gehäuses mit einem oder mehreren Sperrmechanismus/en (141, 142) versehen ist, der/die zum Sperren der ersten und zweiten Partie in der geschlossenen Position angepasst ist/sind, wobei der eine oder die mehreren Sperrmechanismus/en dazu angeordnet ist/sind, durch einen Nutzer von der Außenseite des Gehäuses betrieben zu werden, wenn das Gehäuse in der geschlossenen Position ist.

3. Vorrichtung gemäß Anspruch 1 oder 2, die ferner ein Vorspannbauteil (45) zum Vorspannen der zweiten Partie zu der offenen Position aufweist.

4. Vorrichtung gemäß einem der vorangehenden Ansprüche, wobei die erste Partie (110) eine Innenfläche (111) hat und wobei die zweite Partie (120) eine Innenfläche (121) hat, wobei eine Schicht eines flüssigkeitsabsorbierenden Materials (115, 116) an der Innenfläche (111, 121) der ersten und/oder zweiten Partie vorgesehen ist, zum Verhindern, dass eine Flüssigkeit, die einen medizinisch aktiven Inhaltsstoff enthält, entlang der Innenfläche der ersten und/oder zweiten Partie aus der Vorrichtung läuft.

5. Vorrichtung gemäß einem der vorangehenden Ansprüche, wobei die Pumpe (20) dazu angepasst ist, durch Pressen des Behälters (10) relativ zu der ersten und zweiten Gehäusepartie in Richtung der Spraydispensieröffnung (21) betätigt zu werden, vorzugsweise durch Pressen des Behälters in Richtung der Öffnung entlang der Drehachse.

6. Vorrichtung gemäß einem der vorangehenden Ansprüche, wobei der Behälter (10) einen Umfangsabschnitt (11), der innerhalb des Gehäuses angeordnet ist, und eine Endfläche (12) aufweist, die distal von der Dispensieröffnung beabstandet ist und dazu angeordnet (12) ist, durch einen Nutzer zum Betätigen der Pumpe in Richtung der Spraydispensieröffnung gepresst zu werden, vorzugsweise wobei die Endfläche außerhalb des Gehäuses angeordnet ist.

7. Vorrichtung gemäß Anspruch 6, wobei der Umfangsabschnitt (11) des Behälters ein transparentes Material aufweist und wobei die erste und/oder zweite Partie des Gehäuses einen durchsichtigen Abschnitt (119) aufweist, zum Ermöglichen einer visuellen Inspektion durch den durchsichtigen Abschnitt und das transparente Material eines Flüssigkeitsstands in dem Behälter (10), wenn das Gehäuse (100) in der geschlossenen Position ist.

8. Vorrichtung gemäß einem der vorangehenden Ansprüche, wobei der flexible Kragen (131) eine Umfangsdistalkante (133) aufweist, die dazu geformt und angepasst ist, entlang der gesamten Kante gegen eine Achselhöhle anzuliegen, wenn die zweite Partie in der offenen Position ist, vorzugsweise wobei die Distalkante dazu angepasst ist, sich auf sich selbst zu falten, wenn die zweite Partie in der geschlossenen Position ist, zum Dichten eines Innenraums des Gehäuses (100).

9. Vorrichtung gemäß Anspruch 8, wobei, wenn in einer Projektion auf eine zu der Sprühachse (S) normale und zu der Drehachse (R) parallele Ebene betrachtet und wenn die zweite Partie (120) in der offenen Position ist, jede Partie der Umfangsdistalkante (133) von der Sprühachse (S) um mindestens 3 cm beabstandet ist.

10. Vorrichtung gemäß einem der vorangehenden Ansprüche, die ferner einen Blockiermechanismus (17, 47) aufweist, der zum Verhindern einer Betätigung der Pumpe (20), wenn die zweite Partie (120) in der geschlossenen Position ist, angepasst ist.

11. Vorrichtung gemäß einem der vorangehenden Ansprüche, wobei die Pumpe (20) lösbar innerhalb des Gehäuses (100) angeordnet ist, zum Ermöglichen, dass die Pumpe (20) und/oder die Pumpe und der damit verbundene Behälter (10) ersetzt werden.

12. Vorrichtung gemäß Anspruch 11, die eine betreibbare Verriegelung (118) aufweist, die zwischen einer ersten Position, bei der die Verriegelung eine Bewegung der Pumpe (20) aus dem Gehäuse blockiert, und einer zweiten Position bewegbar ist, bei der die Pumpe von dem Gehäuse in einer Richtung parallel zu der Drehachse entfernt werden kann, wobei die Verriegelung (118) dazu angepasst ist, nur dann von der ersten zu der zweiten Position bewegbar zu sein, wenn das Gehäuse in der geschlossenen Position ist.

13. Vorrichtung gemäß einem der vorangehenden Ansprüche, wobei der Behälter (10) eine wässrige Rezeptur enthält, die ein anticholinergisches Mittel als den aktiven Inhaltsstoff aufweist, wie beispielsweise Tiotropiumbromid, Ipratropium, Glycopyrronium und/oder Oxybutynin.

14. Vorrichtung gemäß einem der vorangehenden Ansprüche, wobei die Drehachse (R) im Wesentlichen parallel zu der Längsachse des Behälters ist, vorzugsweise wobei die Drehachse und die Längsachse des Behälters im Wesentlichen zusammenfallen.

15. Vorrichtung gemäß einem der vorangehenden Ansprüche, wobei die erste Gehäusepartie (110) und die zweite Gehäusepartie (120) jeweils eine Distalkante (113, 123) umfassen, wobei, wenn die zweite Partie in der geschlossenen Position ist, die Distalkanten der ersten und zweiten Partie durch eine erste Entfernung beabstandet sind und, wenn die zweite Partie in der offenen Position ist, die Distalkanten der ersten und zweiten Partie durch eine zweite Entfernung beabstandet sind, die größer ist als die erste Entfernung, vorzugsweise wobei, wenn in einer Projektion auf eine Ebene, die zu der Drehachse normal ist, betrachtet, der Behälter einen Außendurchmesser hat, der größer ist als die erste Entfernung und kleiner ist als die zweite Entfernung.

## Revendications

1. Dispositif de distribution par pulvérisation axillaire portable (1), comprenant :
un récipient (10) destiné à contenir un ingrédient médicalement actif ;
une pompe actionnable (20) connectée au récipient et comprenant un orifice de distribution de pulvérisation (21) avec un axe de pulvérisation (S), dans lequel la pompe est adaptée pour, lors de l'actionnement, libérer une pulvérisation comprenant l'ingrédient médicalement actif à travers ledit orifice le long de l'axe de pulvérisation ;
un boîtier (100) comprenant une première partie (110), une seconde partie (120) et une enveloppe (130) fixée à la première et à la seconde partie, dans lequel la pompe (20) est agencée à l'intérieur du boîtier (100), et dans lequel la première partie et/ou la seconde partie est munie d'une ouverture (O) alignée avec l'orifice de distribution de pulvérisation pour permettre le passage de la pulvérisation libérée à travers l'orifice de distribution,
**caractérisé en ce que** ladite enveloppe est flexible et **en ce que**
la seconde partie (120) peut tourner autour d'un axe de rotation (R) par rapport à la première partie (110) entre une position ouverte dans laquelle la pulvérisation peut sortir du boîtier, et une position fermée dans laquelle la pulvérisation est empêchée de sortir du boîtier.

2. Dispositif selon la revendication 1, dans lequel la première partie (110) et/ou la seconde partie (120) du boîtier est munie d'un ou plusieurs mécanismes de verrouillage (141, 142) adaptés pour verrouiller les première et seconde parties dans la position fermée, dans lequel lesdits un ou plusieurs mécanismes de verrouillage sont agencés pour être actionnés par un utilisateur depuis le côté extérieur du boîtier lorsque le boîtier est dans la position fermée.

3. Dispositif selon la revendication 1 ou 2, comprenant en outre un élément de sollicitation (45) pour solliciter la seconde partie vers la position ouverte.

4. Dispositif selon l'une quelconque des revendications précédentes, dans lequel la première partie (110) présente une surface intérieure (111) et dans lequel la seconde partie (120) présente une surface intérieure (121), dans lequel une couche de matériau absorbant les liquides (115, 116) est prévue sur la surface intérieure (111, 121) de la première et/ou de la seconde partie, pour empêcher un liquide contenant un ingrédient médicalement actif de s'écouler le long de la surface intérieure de la première et/ou de la seconde partie hors du dispositif.

5. Dispositif selon l'une quelconque des revendications précédentes, dans lequel la pompe (20) est adaptée pour être actionnée en pressant le récipient (10) par rapport aux première et seconde parties de boîtier vers l'orifice de distribution de pulvérisation (21), de préférence en pressant le récipient vers ledit orifice le long de l'axe de rotation.

6. Dispositif selon l'une quelconque des revendications précédentes, dans lequel le récipient (10) comprend une partie circonférentielle (11) agencée à l'intérieur du boîtier, et une surface d'extrémité (12) espacée distalement de l'orifice de distribution et agencée (12) pour être pressée vers l'orifice de distribution de pulvérisation par un utilisateur pour actionner la pompe, de préférence dans lequel la surface d'extrémité est agencée à l'extérieur du boîtier.

7. Dispositif selon la revendication 6, dans lequel la partie circonférentielle (11) du récipient comprend un matériau transparent, et dans lequel la première et/ou la seconde partie du boîtier comprend une partie transparente (119) pour permettre une inspection visuelle, à travers la partie transparente et le matériau transparent, d'un niveau de liquide dans le récipient (10) lorsque le boîtier (100) est dans la position fermée.

8. Dispositif selon l'une quelconque des revendications précédentes, dans lequel l'enveloppe flexible (131) comprend un bord distal circonférentiel (133) formé et adapté pour reposer contre une aisselle le long dudit bord entier lorsque la seconde partie est dans la position ouverte, de préférence dans lequel ledit bord distal est adapté pour se replier sur lui-même lorsque la seconde partie est dans la position fermée, pour sceller un espace intérieur du boîtier (100).

9. Dispositif selon la revendication 8, dans lequel lorsqu'il est vu en projection sur un plan perpendiculaire à l'axe de pulvérisation (S) et parallèle à l'axe de rotation (R), et lorsque la seconde partie (120) est dans la position ouverte, chaque partie du bord distal circonférentiel (133) est espacée de l'axe de pulvérisation (S) d'au moins 3 cm.

10. Dispositif selon l'une quelconque des revendications précédentes, comprenant en outre un mécanisme de blocage (17, 47) adapté pour empêcher un actionnement de la pompe (20) lorsque la seconde partie (120) est dans la position fermée.

11. Dispositif selon l'une quelconque des revendications précédentes, dans lequel la pompe (20) est agencée de manière libérable à l'intérieur du boîtier (100), pour permettre à la pompe (20) et/ou à la pompe et au récipient (10) qui lui sont connectés d'être remplacés.

12. Dispositif selon la revendication 11, comprenant un verrou actionnable (118) mobile entre une première position dans laquelle le verrou bloque le déplacement de la pompe (20) hors du boîtier, et une seconde position dans laquelle la pompe peut être retirée du boîtier dans une direction parallèle à l'axe de rotation, dans lequel le verrou (118) est adapté pour être mobile de la première à la seconde position uniquement lorsque le boîtier est dans la position fermée.

13. Dispositif selon l'une quelconque des revendications précédentes, dans lequel le récipient (10) contient une formulation aqueuse a comprenant un agent anticholinergique en tant qu'ingrédient actif, tel que le bromure de tiotropium, l'ipratropium, le glycopyrronium et/ou l'oxybutynine.

14. Dispositif selon l'une quelconque des revendications précédentes, dans lequel l'axe de rotation (R) est sensiblement parallèle à l'axe longitudinal du récipient, de préférence dans lequel l'axe de rotation et l'axe longitudinal du récipient coïncident sensiblement.

15. Dispositif selon l'une quelconque des revendications précédentes, dans lequel la première partie de boîtier (110) et la seconde partie de boîtier (120) comprennent chacune un bord distal (113, 123), dans lequel lorsque la seconde partie est dans la position fermée, les bords distaux de la première et de la seconde partie sont espacés d'une première distance et lorsque la seconde partie est dans la position ouverte, les bords distaux de la première et de la seconde partie sont espacés d'une seconde distance supérieure à la première distance, de préférence dans lequel, lorsqu'il est vu en projection sur un plan perpendiculaire à l'axe de rotation, le récipient présente un diamètre extérieur qui est supérieur à la première distance et inférieur à la seconde distance.
